# EUROPÄISCHE PATENTSCHRIFT

(11) **EP 1 834 940 B1**
(45) Veröffentlichungstag und Bekanntmachung des Hinweises auf die Patenterteilung: **20.01.2010**
(21) Anmeldenummer: 07003257.8
(22) Anmeldetag: 15.02.2007
(51) Int. Cl.: C07C 7/11, C07C 11/06, C07C 11/08

(54) **Verfahren zur Trennung von Olefinen**
Method for separating olefins
Procédé destiné à la séparation d'oléfines

(30) Priorität: 07.03.2006 DE 102006010519
(43) Veröffentlichungstag der Anmeldung: 19.09.2007
(73) Patentinhaber: Linde AG, 80331 München (DE)
(72) Erfinder: Duc, Tuat Pham, 82377 Penzberg (DE)

(56) Entgegenhaltungen:
- EP-A- 1 378 559
- EP-A1- 0 683 146

## Beschreibung

Die Erfindung betrifft ein Verfahren zur Trennung von Olefinen mit drei oder vier Kohlenstoffatomen in einer Fraktionierstufe einer Anlage zur Herstellung von Olefinen aus kohlenwasserstoffhaltigem Einsatz (Olefinanlage), wobei die längerkettigen Olefine des kohlenwasserstoffhaltigen Einsatzes in ein Gasgemisch von kürzerkettigen Olefinprodukten (Rohgas) umgewandelt werden, welches verdichtet und getrocknet wird.

Olefine können zum Beispiel durch thermisches Spalten in einem Spaltofen (Steamcracker) aus längerkettigen Kohlenwasserstoffen hergestellt werden. Das im Spaltofen erzeugte Olefingas (Rohgas) wird nach Abkühlung und Trocknung in die verschiedenen Olefinanteile fraktioniert. Nach dem Stand der Technik beginnt die Trennsequenz entweder mit einer Fraktionierstufe, in der Olefine mit höchstens zwei Kohlenstoffatomen von Olefinen mit mindestens drei Kohlenstoffatomen getrennt werden (C2/C3-Trennung), oder einer Fraktionierstufe, in der Olefine mit höchstens drei Kohlenstoffatomen von Olefinen mit mindestens vier Kohlenstoffatomen (C3/C4-Trennung) getrennt werden.

Beginnt die Trennsequenz mit einer C2/C3-Trennung wird die entstehende Olefinfraktion mit höchstens zwei Kohlenstoffatomen nach einer katalytischen Hydrierung zum Tieftemperaturteil geleitet. Die Olefinfraktion mit mindestens drei Kohlenstoffatomen wird in einem Depropanizer in eine Fraktion mit drei Kohlenstoffatomen und eine Fraktion mit mindestens vier Kohlenstoffatomen getrennt. Die entstandene Fraktion mit drei Kohlenstoffatomen wird anschließend ebenfalls vor ihrer Weiterverarbeitung katalytisch hydriert.

Beginnt die Trennsequenz mit einer C3/C4-Trennung nach dem Stand der Technik erhält man bei Rohgasdruck eine Olefinfraktion mit höchstens drei Kohlenstoffatomen und eine Olefinfraktion mit mindestens drei Kohlenstoffatomen. Eine scharfe Trennung in eine Fraktion mit höchstens drei Kohlenstoffatomen und eine Fraktion mit mindestens vier Kohlenstoffatomen ist bei vollem Rohgasdruck nicht möglich. Hierfür müsste die Temperatur so weit erhöht werden, dass verstärkt Polymer- und somit eine unerwünschte Belagsbildung auftreten würde. Die Fraktion mit höchstens drei Kohlenstoffatomen wird nach einer katalytischen Hydrierung zu einer C2/C3-Trennung geführt. Die Fraktion mit mindestens drei Kohlenstoffatomen wird in eine Fraktion mit drei Kohlenstoffatomen und in eine Fraktion mit mindestens vier Kohlenstoffatomen getrennt. Die entstandene Olefinfraktion mit drei Kohlenstoffatomen muss anschließend ebenfalls katalytisch hydriert werden.

Man benötigt also in beiden Fällen zwei unabhängige katalytische Hydrierungsstufen mit den entsprechenden Festbettreaktoren und Methanolverdampferkühlung zur Temperatureinstellung bei exothermer Reaktionswärme der katalytischen Hydrierung. Die beiden Reaktoren zur katalytischen Hydrierung mit ihren leistungsfähigen Wärmetauschern belasten den Ausrüstungsumfang und damit die Investitionskosten erheblich.

Verfahren zur C2/C3-Trennung werden in den Europäischen Patentschriften EP0683146 und EP1378559 beschrieben.

Der vorliegenden Erfindung liegt die Aufgabe zugrunde die Investitionskosten der Spaltgasaufbereitung der Olefinanlage zu senken.

Diese Aufgabe wird verfahrensseitig dadurch gelöst, dass die C3/C4-Trennung aus einem bei vollem Rohgasdruck betriebenen Absorber (C4-Absorber) und einer Trennkolonne für Olefine mit höchstens drei Kohlenstoffatomen und Olefine mit mindestens vier Kohlenstoffatomen besteht (Depropanizer), wobei der Depropanizer bei einem Druck zwischen 8 bar und 12 bar betrieben wird. Dadurch kann auf eine katalytische Hydrierungsstufe mit entsprechenden Festbettreaktor verzichtet werden.

Üblicherweise fällt in Olefinanlagen nach dem oben geschilderten Stand der Technik entweder eine separate katalytische Hydrierung der Fraktion mit höchstens zwei Kohlenstoffatomen und der Fraktion mit drei Kohlenstoffatomen oder eine separate katalytische Hydrierung der Fraktion mit höchstens drei Kohlenstoffatomen und der Fraktion mit drei Kohlenstoffatomen an.

Der Grundgedanke der Erfindung besteht darin, die beiden separaten katalytischen Hydrierungen durch eine katalytische Hydrierung der gesamten Fraktion mit höchstens drei Kohlenstoffatomen zu ersetzen. Die Gesamtfraktion der Olefine mit höchstens drei Kohlenstoffatomen wird durch die C3/C4-Trennung bestehend aus dem bei vollem Rohgasdruck arbeitenden C4-Absorber und dem in einem Druckbereich zwischen 8 und 12 bar arbeitenden Depropanizer erzeugt.

Gemäß einer besonders bevorzugten Ausführungsform der Erfindung wird das in der Olefinanlage erzeugte kohlenwasserstoffhaltige Gas (Rohgas) bei vollem Rohgasdruck in die C3/C4-Trennung bestehend aus einem bei vollem Rohgasdruck arbeitenden C4-Absorber und einem in einem Druckbereich zwischen 8 und 12 bar arbeitenden Depropanizer geführt und in eine Fraktion mit höchstens drei Kohlenstoffatomen und eine Fraktion mit mindestens vier Kohlenstoffatomen getrennt. Die Einstellung des Drucks des C4-Absorbers wird dabei erfindungsgemäß durch die entsprechenden Druckstufen der Rohgasverdichtungsstufe vorgenommen und liegt vorzugsweise zwischen 15 und 39 bar. Vorteilhafterweise wird die gesamte Fraktion mit höchstens drei Kohlenstoffen der nächsthöheren Stufe des Rohgasverdichters zugeführt und anschließend zur katalytischen Hydrierung weitergeleitet.

Mit der Erfindung gelingt es insbesondere die Investitionskosten durch den Wegfall einer katalytischen Hydrierungsstufe zu minimieren.

Im Folgenden soll die Erfindung anhand eines Vergleichs des Standes der Technik mit dem in den Figuren schematisch dargestellten Ausführungsbeispiel der Erfindung näher erläutert werden.

Es zeigen
- Figur 1: ein Fließschema einer Trennsequenz beginnend mit einer C2/C3- Trennung nach dem Stand der Technik
- Figur 2: ein Fließschema einer Trennsequenz beginnend mit einer C3/C4- Trennung nach dem Stand der Technik
- Figur 3: ein Fließschema der erfindungsgemäße Trennsequenz beginnend mit einer C3/C4-Trennung bestehend aus einem C4-Absorber und einem Depropanizer
- Figur 4: ein Fließschema des C4-Absorbers und des Depropanizers

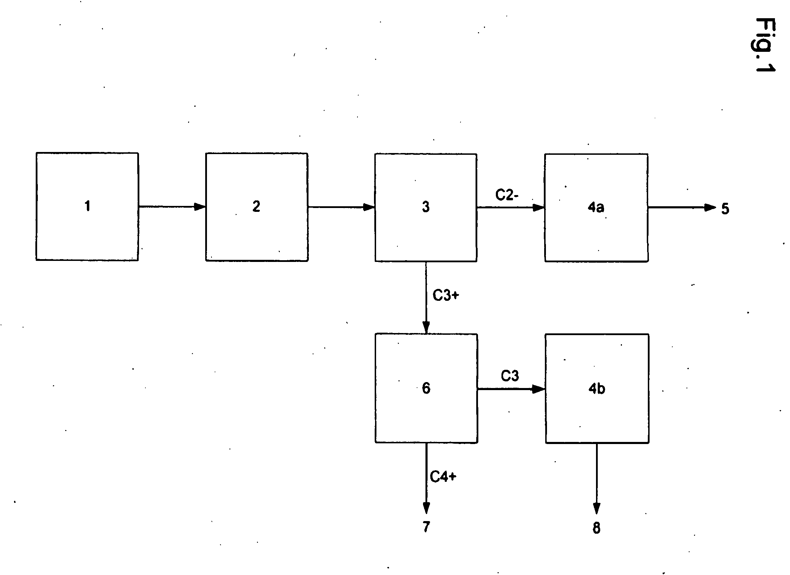

Die in Figur 1 dargestellte Trennsequenz nach dem Stand der Technik beginnt mit der Rohgasverdichtung (1) mit anschließender Vorkühlung und Trocknung (2). Das vorgekühlte und getrocknete Rohgas wird in einer C2/C3-Trennung (3) in Olefine mit höchstens zwei Kohlenstoffatomen (C2-) und Olefine mit mindestens drei Kohlenstoffatomen (C3+) getrennt. Die Olefinfraktion mit höchstens zwei Kohlenstoffatomen wird katalytisch hydriert (4a) und in den Tieftemperaturteil (5) weitergeleitet. Die Olefinfraktion mit mindestens drei Kohlenstoffatomen (C3+) wird unter Druckreduktion in einer C3/C4-Trennung (6) in eine Fraktion mit drei Kohlenstoffatomen (C3) und eine Fraktion mit mindestens vier Kohlenstoffatomen (C4+) getrennt. Die Olefinfraktion mit vier Kohlenstoffatomen wird zur Weiterverarbeitung (7) geführt, während die Fraktion mit drei Kohlenstoffatomen (C3) in einem zweiten Reaktor (4b) katalytisch hydriert und ausgeführt wird (8).

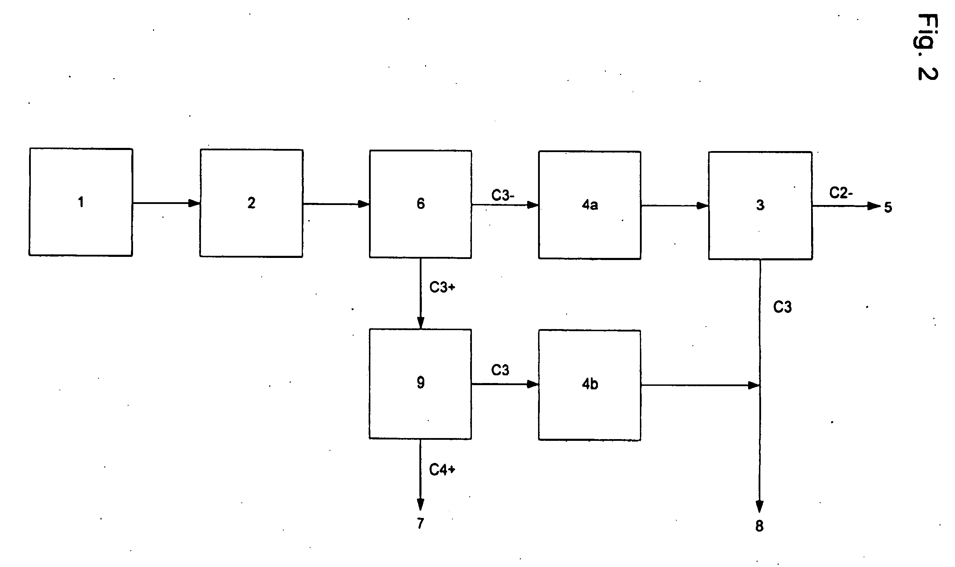

In Figur 2 ist eine Variante des Standes der Technik gezeigt, bei der das vorgekühlte, getrocknete und verdichtete Rohgas (1,2) in eine C3/C4-Trennung (6) geführt wird, wo die Olefine in eine Fraktion mit mindestens drei Kohlenstoffatomen (C3+) und eine Fraktion mit höchstens drei Kohlenstoffatomen (C3-) getrennt werden. Die Fraktion mit mindestens drei Kohlenstoffatomen wird zu einer Fraktionierstufe geführt (9), die Olefine mit höchstens drei Kohlenstoffatomen von Olefinen mit mindestens vier Kohlenstoffatomen trennt. Dadurch entstehen eine Fraktion mit drei Kohlenstoffatomen (C3) und eine Fraktion mit mindestens vier Kohlenstoffatomen (C4+), die zur Weiterverarbeitung ausgeführt wird (7). Die entstandene Fraktion mit drei Kohlenstoffen wird katalytisch hydriert (4b) und ausgeführt (8). Die Fraktion mit höchstens drei Kohlenstoffatomen (C3-) wird in einem Reaktor (4a) katalytisch hydriert. Von dort wird die Olefinfraktion mit höchstens drei Kohlenstoffatomen in eine C2/C3-Trennung (3) geführt und in eine Fraktion mit höchstens zwei Kohlenstoffatomen und eine Fraktion mit drei Kohlenstoffatomen getrennt. Die Olefinfraktion mit höchstens zwei Kohlenstoffatomen wird in den Tieftemperaturteil weitergeleitet (5), während die Fraktion mit drei Kohlenstoffatomen zur Weiterverarbeitung ausgeführt wird (8).

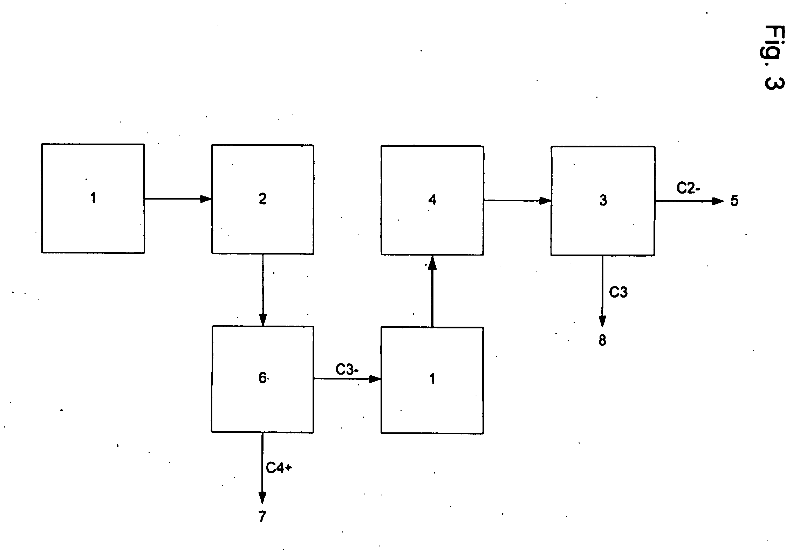

Figur 3 zeigt eine Ausgestaltung der Erfindung. Das Rohgas wird vorverdichtet (1), vorgekühlt und getrocknet (2) und in eine C3/C4-Trennung (6) geführt, die aus einem bei vollem Rohgasdruck arbeitenden C4-Absorber und einem Depropanizer besteht, der bei einem Druck von 8 bis 12 bar betrieben wird. Dort werden die Olefine in eine Fraktion mit höchstens drei Kohlenstoffatomen (C3-) und eine Fraktion mit mindestens vier Kohlenstoffatomen (C4+) getrennt. Die Fraktion mit höchstens drei Kohlenstoffatomen wird vollständig verdichtet (1) und zur katalytischen Hydrierung (4) geführt, während die Fraktion mit mindestens vier Kohlenstoffatomen zur Weiterverarbeitung ausgeführt wird (7). Die Fraktion mit höchstens drei Kohlenstoffatomen wird nach der katalytischen Hydrierung (4) in einer C2/C3-Trennung (3) in eine Fraktion mit mindestens zwei Kohlenstoffatomen (C2-), die zum Tieftemperaturtrennungsteil weitergeleitet wird (5), und eine Fraktion mit drei Kohlenstoffatomen (C3) getrennt. Die Fraktion mit drei Kohlenstoffatomen wird dann zur Weiterverarbeitung geführt (8).

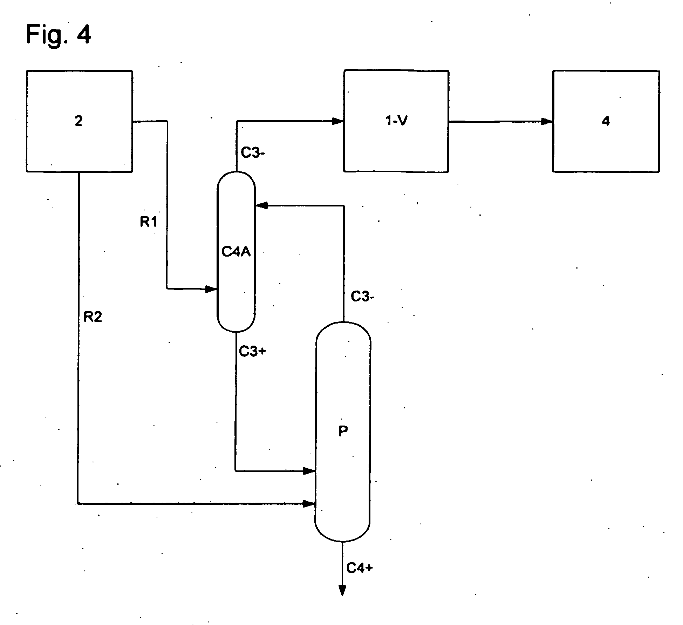

Figur 4 zeigt in einer Ausgestaltung der Erfindung die C3/C4-Trennung im Detail mit Vorkühlung und Trocknung des Rohgases (2). Ein Teil des Rohgases (R1) wird zum C4-Absorber (C4A) geführt und ein zweiter Teil des Rohgases (R2) wird direkt auf den Depropanizer (P) geführt. Am C4-Absorber (C4A) entstehen bei vollem Rohgasdruck eine Fraktion mit mindestens drei Kohlenstoffatomen (C3+) und eine Fraktion mit höchstens drei Kohlenstoffatomen (C3-). Im Depropanizer, der in einem Druckbereich zwischen 8 und 12 bar arbeitet, entstehen eine Fraktion mit höchstens drei Kohlenstoffatomen (C3-) und eine Fraktion mit mindestens vier Kohlenstoffatomen (C4+). Durch die zyklische Führung zwischen C4-Absorber (C4A) und Depropanizer (P) kann das Rohgas komplett in eine Fraktion mit höchstens drei Kohlenstoffatomen (C3-) und eine Fraktion mit mindestens vier Kohlenstoffatomen (C4+) getrennt werden. Die Fraktion mit höchstens drei Kohlenstoffatomen (C3-) wird dann über die letzte Stufe der Verdichtung (1-V) zur katalytischen Hydrierung geführt (4).

## Patentansprüche

1. Verfahren zur Trennung von Olefinen mit drei Kohlenstoffatomen von Olefinen mit vier Kohlenstoffatomen in einer Fraktionierstufe (C3/C4-Trennung) einer Anlage zur Herstellung von Olefinen aus kohlenwasserstoffhaltigem Einsatz (Olefinanlage), wobei die längerkettigen Olefine des kohlenwasserstoffhaltigen Einsatzes in ein Gasgemisch von kürzerkettigen Olefinprodukten (Rohgas) umgewandelt werden, welches verdichtet und getrocknet wird, **dadurch gekennzeichnet, dass** bei der C3/C4-Trennung das Rohgas über einen bei vollem Rohgasdruck betriebenen Absorber (C4-Absorber) und eine Trennkolonne für Olefine mit höchstens drei Kohlenstoffatomen und Olefine mit mindestens vier Kohlenstoffatomen (Depropanizer) geführt wird, wobei der Depropanizer bei einem Druck zwischen 8 bar und 12 bar betrieben wird.

2. Verfahren nach Anspruch 1, **dadurch gekennzeichnet, dass** das Rohgas als Einsatz in den C4-Absorber geführt wird.

3. Verfahren nach Anspruch 1 und 2, **dadurch gekennzeichnet, dass** das Rohgas über den C4-Absorber in eine Fraktion von Olefinen mit höchstens drei Kohlenstoffatomen und eine Fraktion von Olefinen mit mindestens drei Kohlenstoffatomen getrennt wird.

4. Verfahren nach einem der Ansprüche 1 bis 3, **dadurch gekennzeichnet, dass** die Olefinfraktion mit mindestens drei Kohlenstoffatomen durch den Depropanizer in eine Fraktion mit drei Kohlenstoffatomen und eine Fraktion mit mindestens vier Kohlenstoffatomen getrennt wird.

## Claims

1. Method for separating olefins having three carbon atoms from olefins having four carbon atoms in a fractionating stage (C3/C4 separation) of a plant for producing olefins from hydrocarbonaceous feed (olefin plant), wherein the longer-chain olefins of the hydrocarbonaceous feed are converted into a gas mixture of shorter-chain olefin products (crude gas) which is compressed and dried, **characterized in that**, in the C3/C4 separation, the crude gas is passed through an absorber (C4 absorber) operated at full crude gas pressure and a separation column for olefins having at most three carbon atoms and olefins having at least four carbon atoms (depropanizer), wherein the depropanizer is operated at a pressure between 8 bar and 12 bar.

2. Method according to Claim 1, **characterized in that** the crude gas is passed as feed into the C4 absorber.

3. Method according to Claim 1 and 2, **characterized in that** the crude gas is separated via the C4 absorber into a fraction of olefins having at most three carbon atoms and a fraction of olefins having at least three carbon atoms.

4. Method according to any one of Claims 1 to 3, **characterized in that** the olefin fraction having at least three carbon atoms is separated by the depropanizer into a fraction having three carbon atoms and a fraction having at least four carbon atoms.

## Revendications

1. Procédé pour la séparation d'oléfines présentant trois atomes de carbone d'oléfines présentant quatre atomes de carbone dans une étape de fractionnement (séparation C3/C4) d'une installation pour la préparation d'oléfines à partir d'une charge contenant des hydrocarbures (installation d'oléfines), où les oléfines à chaîne plus longue de la charge contenant des hydrocarbures sont transformées dans un mélange gazeux de produits oléfiniques plus courts (gaz brut) qui est comprimé et séché, **caractérisé en ce que** lors de la séparation C3/C4, le gaz brut est guidé sur un absorbant (absorbant C4) opéré à la pression totale du gaz brut et sur une colonne de séparation pour les oléfines présentant au plus trois atomes de carbone et les oléfines présentant au moins quatre atomes de carbone (dépropaniseur), où le dépropaniseur est opéré à une pression entre 8 bars et 12 bars.

2. Procédé selon la revendication 1, **caractérisé en ce que** le gaz brut est guidé comme charge dans l'absorbant C4.

3. Procédé selon la revendication 1 et 2, **caractérisé en ce que** le gaz brut est séparé via l'absorbant C4 en une fraction d'oléfines présentant au plus trois atomes de carbone et une fraction d'oléfines présentant au moins trois atomes de carbone.

4. Procédé selon l'une quelconque des revendications 1 à 3, **caractérisé en ce que** la fraction oléfinique présentant au moins trois atomes de carbone est séparée par le dépropaniseur en une fraction présentant trois atomes de carbone et une fraction présentant au moins quatre atomes de carbone.
